# EUROPÄISCHE PATENTSCHRIFT

(11) **EP 0 976 725 B1**
(45) Veröffentlichungstag und Bekanntmachung des Hinweises auf die Patenterteilung: **03.03.2004**
(21) Anmeldenummer: 99114114.4
(22) Anmeldetag: 16.07.1999
(51) Int. Cl.: C07C 319/20

(54) **Verfahren zur kontinuierlichen Herstellung von Amino-halogenphenyl-alkylthioethern**
Process for the continuous preparation of amino-halogenophenyl alkyl thioethers
Procédé pour la préparation continue d'amino- halogéno-phényl alkyl thioéthers

(30) Priorität: 29.07.1998 DE 19834102
(43) Veröffentlichungstag der Anmeldung: 02.02.2000
(73) Patentinhaber: Bayer Chemicals AG, 51368 Leverkusen (DE)
(72) Erfinder: Kiel, Wolfgang, Dr., 51519 Odenthal (DE)

(56) Entgegenhaltungen:
- EP-A- 0 315 862
- EP-A- 0 331 144
- EP-A- 0 775 693
- WO-A-96/11906
- INOUE H ET AL: "SYNTHESIS OF HALOGEN-SUBSTITUTED 1,5-BENZOTHIAZEPINE DERIVATIVES AND THEIR VASODILATING AND HYPOTENSIVE ACTIVITIES" JOURNAL OF MEDICINAL CHEMISTRY, AMERICAN CHEMICAL SOCIETY. WASHINGTON, US, Bd. 34, 1. Februar 1991 (1991-02-01), Seiten 675-687, XP000616258 ISSN: 0022-2623
- KATSUMI ITOH ET AL.: "Synthesis and angiotensin converting enzyme inhibitory activity of 1,5-benzothiazepine and 1,5-benzoxazepine derivatives." CHEMICAL AND PHARMACEUTICAL BULLETIN., Bd. 34, Nr. 3, 1986, Seiten 1128-1147, XP001030863 PHARMACEUTICAL SOCIETY OF JAPAN. TOKYO., JP ISSN: 0009-2363
- CORRAL C ET AL: "SYNTHESIS OF 10-(4-METHYL-1-PIPERAZINYL)THIENOU3,2-BU1, 5BENZOTHIAZEPINES" JOURNAL OF HETEROCYCLIC CHEMISTRY, HETEROCORPORATION. PROVO, US, Bd. 22, 1. September 1985 (1985-09-01), Seiten 1345-1348, XP000616259 ISSN: 0022-152X

## Beschreibung

Die vorliegende Erfindung betrifft ein kontinuierliches Verfahren zur Herstellung von Amino-halogenphenyl-alkylthioethern durch selektive katalytische Hydrierung der entsprechenden Nitro-Verbindungen.

Amino-halogenphenyl-alkylthioether sind Zwischenprodukte zur Herstellung von Pflanzenschutzmitteln (siehe z.B.EP-A 306 222).

Bei den Einsatzmaterialien ist unter den Bedingungen einer katalytischen Hydrierung mit der Abspaltung von Halogenatomen und Alkylmercaptogruppen zu rechnen (siehe z.B. Chem. Ind. 53, 103-109 (1994) und Vergleichsbeispiel 1).

Bekannt ist ein diskontinuierliches Verfahren zur Herstellung von 2-Amino-6-chlorphenyl-alkylthioethern durch katalytische Hydrierung mit einem Raney-Nickel-Katalysator (siehe DE-A 195 43 475). Die Selektivität, die bei diesem diskontinuierlichen Verfahren erreicht wird, verschlechtert sich jedoch deutlich, wenn man versucht, es kontinuierlich durchzuführen (siehe Vergleichsbeispiel 1). Die zunehmende Abspaltung von Chlor und Alkylmercaptogruppen führt außerdem zu einem vorzeitigen Aktivitätsverlust des Raney-Nickel-Katalysators, so daß er in einem kontinuierlichen Verfahren häufiger ausgetauscht werden müßte, was zusätzlichen Aufwand erfordern würde.

Es wurde nun ein Verfahren zur Herstellung von Amino-halogenphenyl-alkylthioethem durch katalytische Reduktion der entsprechenden Nitroverbindungen gefunden, das dadurch gekennzeichnet ist, daß man die Nitroverbindung in Gegenwart eines Edelmetall-auf-Träger-Katalysators kontinuierlich mit Wasserstoff hydriert.

In das erfindungsgemäße Verfahren können z.B. Nitroverbindungen der Formel (I) eingesetzt werden 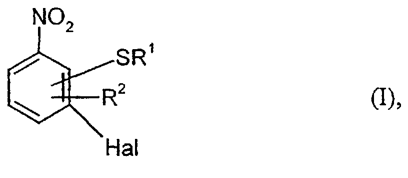 in der
- SR¹: sich in ortho- oder para-Stellung zur Nitrogruppe befindet und
- R¹: für C₁-C₁₈-Alkyl, C₃-C₈-Cycloalkyl oder C₇-C₁₀-Arylalkyl,
- R²: für Wasserstoff, C₁-C₄-Alkyl, C₁-C₄-Alkoxy oder Benzyl und
- Hal: für Chlor oder Brom stehen.

Aus solchen Nitroverbindungen kann man die entsprechenden Amino-halogenphenyl-alkylthioether der Formel (II) erhalten 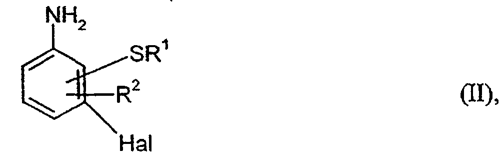 in der
- SR¹: sich in ortho- oder para-Stellung zur Aminogruppe befindet und
- R¹, R²: und Hal die bei Formel (I) angegebene Bedeutung haben.

In den Formeln (I) und (II) befindet sich R² vorzugsweise in ortho-Stellung zu Hal. Beispiele für C₁-C₄-Alkyl sind Methyl, Ethyl, n-Propyl, i-Propyl, n-Butyl, i-Butyl und tert.-Butyl.

Beispiele für C₁-C₁₈-Alkyl sind zusätzlich die isomeren Amyle, Hexyle, Octyle, Decyle, Dodecyle, Heptadecyle und Octadecyle.

Beispiele für C₃-C₈-Cycloalkyl sind Cyclopropyl, Cyelobutyl, Cyclopentyl, Cyclohexyl, Cycloheptyl, Methylcyclopropyl, Dimethylcyclopropyl, Methylcyclopentyl, Dimethylcyclopentyl, Methylcyclohexyl und Dimethylcyclohexyl.

Beispiele für C₇-C₁₀-Arylalkylgruppen sind Benzyl, α- und β-Phenylethyl, Phenylpropyl und Phenylbutyl.

Beispiele für C₁-C₄-Alkoxygruppen sind Methoxy, Ethoxy, Propoxy, n-Butoxy und i-Butoxy.

Bevorzugte Alkylgruppen sind Methyl, Ethyl und Isopropyl, bevorzugte Cycloalkylgruppen sind Cyclopropyl, Cyclopentyl und Cyclohexyl sowie die entsprechenden Methyl- und Dimethylcycloalkyle, bevorzugte Arylalkylgruppen sind Benzyl und Phenylethyl und bevorzugte Alkoxygruppen sind Methoxy und Ethoxy.

R¹ steht vorzugsweise für eine der als bevorzugt bezeichneten Alkylgruppen und R² steht vorzugsweise für Wasserstoff oder eine der als bevorzugt bezeichneten Alkylgruppen.

Besonders bevorzugte Nitroverbindungen der Formel (I) sind 2-Chlor-6-nitrophenylmethylthioether, 2-Chlor-6-nitrophenyl-isopropylthioether, 2-Fluor-6-nitrophenylisopropylthioether, 4-Chlor-6-nitrophenyl-isopropylthioether, 4-Fluor-6-nitrophenylisopropylthioether, 2-Chlor-4-nitrophenyl-isopropylthioether und 2-Fluor-4-nitrophenyl-isopropylthioether, aus denen man die entsprechenden Aminoverbindungen der Formel (II) erhält.

Das erfindungsgemäße Verfahren wird i.a. in Gegenwart eines Lösungsmittels durchgeführt. In Frage kommen beispielsweise Alkohole, Alkane, Cycloalkane und Aromaten. Bevorzugt sind Methanol, Ethanol, Isopropanol, Methylcyclohexan und Toluol. Man kann auch beliebige Mischungen dieser Lösungsmittel einsetzen. Die eingesetzte Nitroverbindung und die erhaltene Aminoverbindung muß sich im jeweiligen Lösungsmittel nicht unbedingt vollständig lösen. Es genügt wenn ein Teil davon gelöst ist und der Rest in suspendierter Form vorliegt. Bevorzugt setzt man Methanol, Ethanol, Isopropanol, Methylcyclohexan und/oder Toluol in solchen Mengen ein, daß die eingesetzten Nitroverbindungen und die erhaltenen Aminoverbindungen vollständig gelöst vorliegen können.

Es ist ein wesentliches Merkmal der vorliegenden Erfindung zur kontinuierlichen Her-stellung von Amino-halogenphenyl-alkylthioethem, daß ein Edelmetall-auf-Träger-Katalysator zum Einsatz kommt. Bei den Edelmetallen kann es sich beispielsweise um Palladium oder Platin handeln, bei den Trägermaterialien beispielsweise um Aluminiumoxide, Kieselsäuren oder Kohlen der verschiedensten Art (etwa Holzkohle, Knochenkohle oder Aktivkohle) handeln. Bevorzugt sind Platin-auf-Kohle-Katalysatoren. Die Trägerkatalysatoren können z.B. 0,1 bis 10 Gew.-%, vorzugsweise 1 bis 8 Gew.-% Edelmetall enthalten.

Weiterhin ist es bevorzugt, die Edelmetall-auf-Träger-Katalysatoren, insbesondere Platin-auf-Kohle-Katalysatoren in sulfidierter Form einzusetzen. Häufig fallen dann die Amino-halogenphenyl-alkylthioether in noch besseren Selektivitäten an. Sulfidierte Edelmetall-auf-Träger-Katalysatoren können z.B. 0,001 bis 1 Gew.-% Schwefel (bezogen auf das Edelmetall) enthalten. Vorzugsweise liegt diese Menge bei 0,005 bis 0,5 Gew.-%.

Für das erfindungsgemäße Verfahren geeignete Katalysatoren, auch in sulfidierter Form, sind im Handel erhältlich.

Die Menge Katalysator, bezogen auf die eingesetzte Nitroverbindung, kann in weiten Grenzen variiert werden. Beispielsweise kann man bezogen auf einen Durchsatz an Nitroverbindung von 1 mol pro Stunde 3 bis 30 g Katalysator (Summe Edelmetall + Träger) im Reaktionsgefäß vorliegen haben. Vorzugsweise liegt diese Menge bei 5 bis 15 g Katalysator.

Das erfindungsgemäße Verfahren kann z.B. bei Temperaturen im Bereich 20 bis 150°C und z.B. Drucken im Bereich von Normaldruck bis 300 bar durchgeführt werden. Bevorzugt sind 30 bis 120°C und 10 bis 150 bar.

Zur Durchführung des erfindungsgemäßen Verfahren kann man beispielsweise Reaktoren verwenden, mit denen sich Verweilzeiten von beispielsweise 10 bis 400 Minuten realisieren lassen. Vorzugsweise beträgt die Verweilzeit 30 bis 180 Minuten.
Geeignete Reaktortypen sind z.B.gerührte Autoklaven, Schlaufenreaktoren und Rohrreaktoren.

Zur Abtrennung und Isolierung der hergestellten Amino-halogenphenyl-alkylthioether kann man auf verschiedene Weise verfahren. Beispielsweise kann man dem Reaktor ausreagiertes Reaktionsgemisch in einer Menge entnehmen, die dem Zulauf entspricht. Aus dem entnommenen Reaktionsgemisch kann man den Katalysator (z.B. durch Filtration) und das Lösungsmittel (z.B. durch Destillation) abtrennen und beides zurückführen. Man kann den Reaktor auch mit Einrichtungen versehen, die ein Austragen des Katalysators verhindern und nur flüssige Anteile des ausreagierten Reaktionsgemisches entnehmen und aufarbeiten. Aus Gemischen, die im wesentlichen Lösungsmittel und hergestelltes Produkt enthalten kann man das hergestellte Produkt gegebenenfalls auch durch Abkühlen kristallisieren und dann z.B. durch Filtration abtrennen. Es sind auch noch andere Aufarbeitungs- und Isolierungsmethoden denkbar.

Es ist vorteilhaft, den Katalysator von Zeit zu Zeit, beispielsweise wenn seine Aktivität nach beispielsweise 2 bis 10 Tagen Laufzeit nachgelassen hat, ganz oder teilweise auszuschleusen und durch frischen Katalysator zu ersetzen. Die hergestellten Amino-halogenphenyl-alkylthioether liegen nach ihrer Abtrennung und Isolierung im allgemeinen bereits in einer Reinheit von 99 % oder höher vor.

Das erfindungsgemäße Verfahren hat den Vorteil, daß mit ihm Amino-halogenphenyl-alkylthioether kontinuierlich in hohen Reinheiten und mit geringer Nebenproduktbildung (z.B. durch Abspaltung von Chlor und/oder Alkylthiogruppen verursacht) hergestellt werden können. Dies ist besonders überraschend, denn Edelmetalle sind i.a. gegen Schwefelverbindungen empfindlicher als Raney-Nickel. Häufig desaktivieren Schwefelverbindungen Edelmetallkatalysatoren. Hier tritt aber der umgekehrte Effekt auf.

### Beispiele

### Beispiel 1

80 g 2-Chlor-6-nitrophenyl-isopropylthioether, 320 g Methanol und 9,6 g Katalysator (Platin-auf-Kohle, 5 gew.-%ig) wurden in einen 0,7 l-Autoklaven vorgelegt. Nach dem Spülen mit Stickstoff wurden 50 bar Wasserstoff aufgepreßt und auf 80°C erhitzt. Nach dem Anspringen der Hydrierung wurde der Wasserstoffdruck auf 80 bar erhöht und auf 80 bar gehalten. In das ausreagierte Reaktionsgemisch wurde nun kontinuierlich ein Gemisch der o.a. Zusammensetzung hineingepumpt und kontinuierlich ausreagiertes Reaktionsgemisch in gleicher Menge entnommen. Aus dem ausgeschleusten Reaktionsgemisch wurde der Katalysator durch Filtration und Methanol durch Destillation abgetrennt und beides in die Reaktion zurückgeführt.

Bei einer Verweilzeit im Reaktor von 30 Minuten wurde so 2-Amino-6-chlorphenylisopropylthioether mit einer Reinheit von 99,28 % (GC) erhalten. Chlorfreie Aromaten hatten sich nur zu weniger als 0,1 % und von Isopropylmercaptogruppen freie Aromaten ebenfalls nur zu weniger als 0,1 % gebildet.

### Beispiel 2

Es wurde verfahren wie in Beispiel 1 beschrieben, jedoch wurden 9,6 g eines sulfidierten Katalysators der Fa. Engelhard eingesetzt, der 3 Gew.-% Platin-auf-Kohle enthielt.

Bei einer Verweilzeit im Reaktor von 20 Minuten wurde so 2-Amino-6-chlorphenylisopropylthioether mit einer Reinheit von 99,61 % (GC) erhalten. Chlorfreie Aromaten und von Isopropylmercaptogruppen freie Aromaten hatten sich je nur zu weniger als 0,1 % gebildet.

### Vergleichsbeispiel 1

Es wurde verfahren wie in Beispiel 1 beschrieben, jedoch wurden als Katalyator 6 g Raney-Nickel des Typs Ra Ni37S (Fa. Bayer) eingesetzt.

Bei einer Verweilzeit im Reaktor von 60 Minuten wurde so 2-Amino-6-chlorphenylisopropylthioether mit einer Reinheit von 97,33 % (GC) erhalten. Chlorfreie Aromaten hatten sich zu 2,1 % und von Isopropylymercaptogruppen freie Aromaten zu 0,52 % gebildet.

## Patentansprüche

1. Verfahren zur Herstellung von Amino-halogenphenyl-alkylthioethern durch katalytische Reduktion der entsprechenden Nitroverbindungen, **dadurch gekennzeichnet, daß** man die Nitroverbindung in Gegenwart eines Edelmetall-auf-Träger-Katalysators kontinuierlich mit Wasserstoff hydriert.

2. Verfahren nach Anspruch 1, **dadurch gekennzeichnet, daß** man als Nitroverbindungen solche der Formel (I) einsetzt 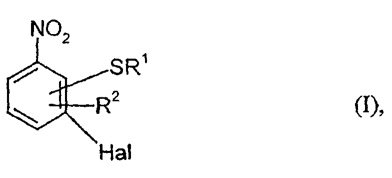 in der
SR¹ sich in ortho- oder para-Stellung zur Nitrogruppe befindet und
R¹ für C₁-C₁₈-Alkyl, C₃-C₈-Cycloalkyl oder C₇-C₁₀-Arylalkyl,
R² für Wasserstoff, C₁-C₄-Alkyl, C₁-C₄-Alkoxy oder Benzyl und
Hal für Chlor oder Brom stehen,
und Amino-halogenphenyl-alkylthioether der Formel (II) erhält 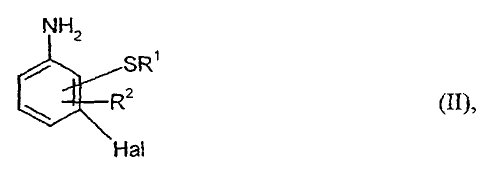 in der
SR¹ sich in ortho- oder para-Stellung zur Aminogruppe befindet und
R¹, R² und Hal die bei Formel (I) angegebene Bedeutung haben.

3. Verfahren nach Ansprüchen 1 und 2, **dadurch gekennzeichnet, daß** man 2-Chlor-6-nitrophenyl-methylthioether, 2-Chlor-6-nitrophenyl-isopropylthioether, 2-Fluor-6-nitrophenyl-isopropylthioether, 4-Chlor-6-nitrophenyl-isopropylthioether, 4-Fluor-6-nitrophenyl-isopropylthioether, 2-Chlor-4-nitrophenyl-isopropylthioether und 2-Fluor-4-nitrophenyl-isopropylthioether einsetzt und die entsprechenden Aminoverbindungen erhält.

4. Verfahren nach Ansprüchen 1 bis 3, **dadurch gekennzeichnet, daß** man in Gegenwart von Alkoholen, Alkanen, Cycloalkanen oder Aromaten als Lösungsmittel arbeitet.

5. Verfahren nach Ansprüchen 1 bis 4, **dadurch gekennzeichnet, daß** man als Katalysatoren Palladium oder Platin-auf-Kohle-Katalysatoren einsetzt.

6. Verfahren nach Ansprüchen 1 bis 5, **dadurch gekennzeichnet, daß** man sulfidierte Edelmetall-auf-Träger-Katalysatoren einsetzt.

7. Verfahren nach Ansprüchen 1 bis 6, **dadurch gekennzeichnet, daß** man bezogen auf einen Durchsatz an Nitroverbindung von 1 mol/h 3 bis 30 g Katalysator (Summe Edelmetall + Träger) im Reaktionsgefäß vorliegen hat.

8. Verfahren nach Ansprüchen 1 bis 7, **dadurch gekennzeichnet, daß** man es bei Temperaturen im Bereich 20 bis 150°C und Drucken im Bereich von Normaldruck bis 300 bar durchführt.

9. Verfahren nach Ansprüchen 1 bis 8, **dadurch gekennzeichnet, daß** man Reaktoren verwendet, mit denen sich Verweilzeiten von 10 bis 400 Minuten realisieren lassen.

10. Verfahren nach Ansprüchen 1 bis 9, **dadurch gekennzeichnet, daß** man dem Reaktor ausreagiertes Reaktionsgemisch in einer Menge entnimmt, die dem Zulauf entspricht und daraus den Katalysator und das Lösungsmittel abtrennt und zurückführt.

## Claims

1. Process for the preparation of amino-halogenophenyl alkyl thioethers by catalytic reduction of the corresponding nitro compounds, **characterized in that** the nitro compound is continuously hydrogenated with hydrogen in the presence of a supported noble-metal catalyst.

2. Process according to Claim 1, **characterized in that** the nitro compounds used are those of the formula (I) 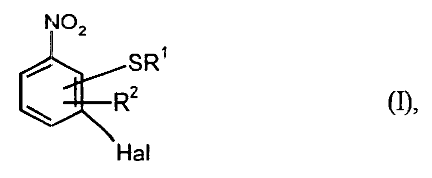 where
SR¹ is in the ortho- or para-position relative to the nitro group and
R¹ is C₁-C₁₈-alkyl, C₃-C₈-cycloalkyl or C₇-C₁₀-arylalkyl,
R² is hydrogen, C₁-C₄-alkyl, C₁-C₄-alkoxy or benzyl, and
Hal is chlorine or bromine,
and amino-halogenophenyl alkyl thioethers of the formula (II) are obtained 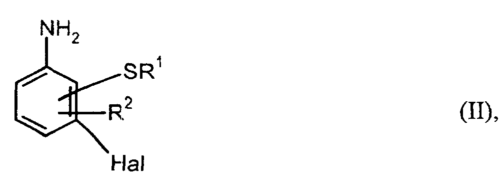 where
SR¹ is in the ortho- or para-position relative to the amino group, and
R¹, R² and Hal are as defined for formula (I).

3. Process according to Claims 1 and 2, **characterized in that** 2-chloro-6-nitrophenyl methyl thioether, 2-chloro-6-nitrophenyl isopropyl thioether, 2-fluoro-6-nitrophenyl isopropyl thioether, 4-chloro-6-nitrophenyl isopropyl thioether, 4-fluoro-6-nitrophenyl isopropyl thioether, 2-chloro-4-nitrophenyl isopropyl thioether and 2-fluoro-4-nitrophenyl isopropyl thioether are used and the corresponding amino compounds are obtained.

4. Process according to Claims 1 to 3, **characterized in that** it is carried out in the presence of alcohols, alkanes, cycloalkanes or aromatics as solvents.

5. Process according to Claims 1 to 4, **characterized in that** the catalysts are palladium or platinum-on-charcoal catalysts.

6. Process according to Claims 1 to 5, **characterized in that** sulphidized supported noble-metal catalysts are used.

7. Process according to Claims 1 to 6, **characterized in that**, based on a throughput of nitro compound of 1 mol/h, from 3 to 30 g of catalyst (total of noble metal + support) are present in the reaction vessel.

8. Process according to Claims 1 to 7, **characterized in that** it is carried out at temperatures in the range from 20 to 150°C and pressures in the range from atmospheric pressure to 300 bar.

9. Process according to Claims 1 to 8, **characterized in that** the reactors used are those with which residence times of from 10 to 400 minutes can be achieved.

10. Process according to Claims 1 to 9, **characterized in that** fully reacted reaction mixture is removed from the reactor in an amount corresponding to the feed, and the catalyst and the solvent are removed therefrom and recycled.

## Revendications

1. Procédé de préparation d'aminohalogénophényl-alkylthioéthers par réduction catalytique des composés nitrés correspondants, **caractérisé en ce que** le composé nitré est hydrogéné en continu avec de l'hydrogène en présence d'un catalyseur de métal noble sur support.

2. Procédé selon la revendication 1, **caractérisé en ce que** des composés nitrés de formule (I) 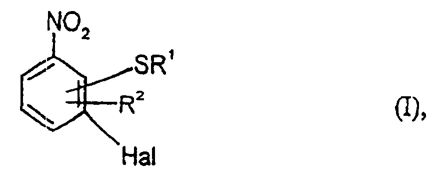 dans laquelle
SR¹ se trouve en position ortho ou para par rapport au radical nitro et
R¹ désigne un radical alkyle C₁-C₁₈, cycloalkyle C₃-C₈ ou arylalkyle C₇-C₁₀,
R² désigne l'hydrogène, un radical alkyle C₁-C₄, alcoxy C₁-C₄ ou benzyle et
Hal désigne le chlore ou le brome
sont utilisés et des aminohalogénophényl-alkylthioéthers de formule (II) 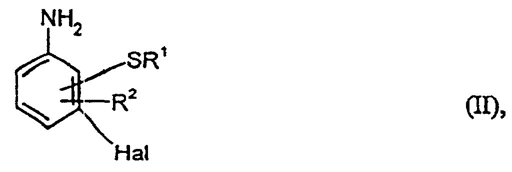 dans laquelle
SR¹ se trouve en position ortho ou para par rapport au radical amino et
R¹, R² et Hal ont la signification spécifiée dans la formule (I)
sont obtenus.

3. Procédé selon l'une des revendications 1 et 2, **caractérisé en ce que** le 2-chloro-6-nitrophényl-méthylthioéther, le 2-chloro-6-nitrophényl-isopropylthioéther, le 2-fluoro-6-nitrophényl-isopropylthioéther, le 4-chloro-6-nitrophényl-isopropylthioéther, le 4-fluoro-6-nitrophényl-isopropylthioéther, le 2-chloro-4-nitrophényl-isopropylthioéther et le 2-fluoro-4-nitrophényl-isopropylthioéther sont utilisés et les composés aminés correspondants sont obtenus.

4. Procédé selon l'une des revendications 1 à 3, **caractérisé en ce que** l'on procède en présence d'alcools, d'alcanes, de cycloalcanes ou d'aromatiques comme solvants.

5. Procédé selon l'une des revendications 1 à 4, **caractérisé en ce que** des catalyseurs de palladium ou de platine sur charbon sont utilisés comme catalyseurs.

6. Procédé selon l'une des revendications 1 à 5, **caractérisé en ce que** des catalyseurs de métal noble sulfuré sur charbon sont utilisés.

7. Procédé selon l'une des revendications 1 à 6, **caractérisé en ce que** 3 à 30 g de catalyseur (somme du métal noble + du support) sont présents dans le réacteur par rapport à un débit de composé nitré de 1 mole/h.

8. Procédé selon l'une des revendications 1 à 7, **caractérisé en ce que** l'on exécute le procédé à des températures de l'ordre de 20 à 150°C et des pressions de l'ordre de la pression normale à 300 bars.

9. Procédé selon l'une des revendications 1 à 8, **caractérisé en ce que** des réacteurs avec lesquels des temps de séjour de 10 à 400 minutes peuvent être réalisés sont utilisés.

10. Procédé selon l'une des revendications 1 à 9, **caractérisé en ce que** le mélange réactif qui a réagi est prélevé du réacteur dans une quantité qui correspond à la quantité amenée et, à partir de là, le catalyseur et le solvant sont séparés et recyclés.
